# EUROPEAN PATENT SPECIFICATION

(11) **EP 2 805 745 B1**
(45) Date of publication and mention of the grant of the patent: **26.04.2017**
(21) Application number: 14168169.2
(22) Date of filing: 13.05.2014
(51) Int. Cl.: A61N 5/10, G01T 1/29, G01T 3/00, G01T 3/06

(54) **Neutron capture therapy apparatus**
Neutronenerfassungstherapievorrichtung
Appareil de thérapie par capture de neutrons

(30) Priority: 22.05.2013 JP 2013108339
(43) Date of publication of application: 26.11.2014
(73) Proprietor: SUMITOMO HEAVY INDUSTRIES, LTD., Tokyo 141-6025 (JP)
(72) Inventor: Eto, Haruhiko, Kanagawa, 237-8555 (JP)
(74) Representative: Louis Pöhlau Lohrentz

(56) References cited:
- WO-A1-2012/014671
- MATSUMOTO T ET AL: "A new monitoring system for the thermal neutron fluence rate and gamma dose rate in boron neutron capture therapy", PHYSICS IN MEDICINE AND BIOLOGY, INSTITUTE OF PHYSICS PUBLISHING, BRISTOL GB, vol. 33, no. 4, 1 April 1988 (1988-04-01), pages 427-435, XP020023328, ISSN: 0031-9155, DOI: 10.1088/0031-9155/33/4/003
- ISHIKAWA M ET AL: "Development of real-time thermal neutron monitor using boron-loaded plastic scintillator with optical fiber for boron neutron capture therapy", APPLIED RADIATION AND ISOTOPES, ELSEVIER, OXFORD, GB, vol. 61, no. 5, 1 November 2004 (2004-11-01), pages 775-779, XP004526447, ISSN: 0969-8043, DOI: 10.1016/J.APRADISO.2004.05.053
- GÃ MEZ F ET AL: "A new active method for the measurement of slow-neutron fluence in modern radiotherapy treatment rooms; New method for neutron measurement in radiotherapy", PHYSICS IN MEDICINE AND BIOLOGY, INSTITUTE OF PHYSICS PUBLISHING, BRISTOL GB, vol. 55, no. 4, 21 February 2010 (2010-02-21), pages 1025-1039, XP020171781, ISSN: 0031-9155

## Description

### BACKGROUND OF THE INVENTION

### Field of the Invention

The present invention relates to a neutron capture therapy apparatus for irradiating a subject with neutron beams.

### Description of Related Art

As a neutron capture therapy to kill cancer cells by irradiation of neutron beams, a boron neutron capture therapy (BNCT) using a boron compound is known. In the boron neutron capture therapy, a drug containing boron is administered to the patient, the boron is accumulated on a part where cancer cells are present, and the part where the boron is accumulated is irradiated with neutron beams to kill cancer cells.

As a neutron capture therapy apparatus used in such a neutron capture therapy, Japanese Unexamined Patent Application Publication No. 2004-233168 discloses a neutron irradiation apparatus for treatment including a neutron emission device that emits neutron beams toward an irradiation target, a vacuum conduit that guides the neutron beams emitted from the neutron emission device to the irradiation target, and a collimator that makes the neutron beams converge to increase the directivity of the neutrons. In the neutron irradiation apparatus for treatment disclosed in Japanese Unexamined Patent Application Publication No. 2004-233168, a test irradiation step of testing the irradiation of neutron beams to the irradiation target is performed. In this test irradiation step, the irradiation target is irradiated with neutron beams on a trial basis, and the actual amount of activation when irradiating neutron beams is measured. Specifically, the test irradiation step is performed by mounting a gold wire for neutron measurement on a surface and in a deep portion of a target part in the irradiation target, pulling out the gold wire after the irradiation of neutron beams for a fixed period of time, and measuring the amount of activation of the gold wire.

When measuring the irradiated neutron beams by mounting a gold wire in the target part of the irradiation target and measuring the amount of activation of the gold wire as described above, it is necessary to irradiate the gold wire with neutron beams for a fixed period of time and then measure the dose of neutron beams from the activated gold wire. Thus, since the dose of neutron beams should be measured afterward, it is not possible to measure the dose of neutron beams during the irradiation of neutron beams. Accordingly, there is a problem in that the neutron dose cannot be measured in real time.

In addition, when using a scintillation detector that emits light by receiving a radiation or an ionization chamber that generates a current by ionizing the gas between electrodes by receiving a radiation, a signal is output by receiving neutron beams. For this reason, it is not possible to measure the neutron beams in real time. In the scintillationdetector, however, there is a problem in that a scintillator, an optical fiber, and the like deteriorate with the irradiation of neutron beams, and accordingly, neutron beam detection efficiency is gradually lowered. In the ionization chamber, there is a problem in that gas between the electrodes deteriorates due to the irradiation of neutron beams or the neutron beam detection efficiency changes when gas pressure changes. If the neutron beam detection efficiency changes as described above, the state of a signal with respect to the amount of neutron beams that are actually irradiated changes gradually. This causes a problem in that the neutron beam measurement accuracy is reduced.

### SUMMARY OF THE INVENTION

In view of such a problem, it is an object of the invention to provide a neutron capture therapy apparatus capable of measuring the neutron dose in real time while suppressing a reduction in the neutron beam measurement accuracy.

According to an aspect of the invention, there is provided a neutron capture therapy apparatus that irradiates a subject with a neutron beam. The neutron capture therapy apparatus includes: a neutron beam generation unit that generates the neutron beam; a neutron beam irradiation unit that irradiates the neutron beam generated by the neutron beam generation unit toward the subject; a real-time dose output unit that includes a neutron beam detection unit, which detects the neutron beam irradiated from the neutron beam irradiation unit and outputs a signal in real time, and a neutron dose output unit, which converts the signal output from the neutron beam detection unit into a neutron dose using a conversion condition set in advance and outputs the neutron dose in real time; and a conversion condition setting unit that sets the conversion condition. The conversion condition setting unit includes a gamma ray detection section that detects a gamma ray emitted from a metal member activated by irradiation of the neutron beam, and modifies the conversion condition based on a signal, which is output from the neutron beam detection unit by irradiation of the neutron beam, and a neutron dose of the neutron beam, which is acquired based on the gamma ray detected by the gamma ray detection section.

According to the neutron capture therapy apparatus of the invention, the neutron beam detection unit detects the neutron beam irradiated from the neutron beam irradiation unit and outputs a signal in real time, and the neutron dose output unit converts the signal into the neutron dose using the conversion condition set in advance and outputs the neutron dose in real time. Thus, due to the real-time dose output unit including the neutron beam detection unit and the neutron dose output unit, the neutron dose can be output in real time when irradiating the neutron beam to perform treatment. The conversion condition setting unit includes the gamma ray detection section that detects gamma rays emitted from the metal member activated by irradiation of the neutron beam, and modifies (updates) the conversion condition based on the signal, which is out put from the neutron beam detection unit that detects the neutron beam, and the neutron dose acquired based on the gamma rays detected by the gamma ray detection section. Thus, by modifying the conversion condition using the signal from the neutron beam detection unit and the neutron dose obtained from the metal member, the signal is corrected into the neutron dose under the modified conversion condition even if the detection efficiency of the neutron beam detection unit that outputs the signal is lowered. Therefore, it is possible to eliminate a difference between the neutron dose actually irradiated and the measured neutron dose. By converting the signal into the neutron dose under the modified conversion condition and outputting the neutron dose, it is possible to avoid a situation where the measurement accuracy of neutron beams is reduced.

In addition, the neutron beam detection unit may be a scintillator that generates a light signal as the signal by irradiation of the neutron beam. Thus, by using the scintillator as the neutron beam detection unit, it is possible to reduce the size of the neutron beam detection unit.

According to an aspect of the disclosure, there is provided a neutron beam measuring method in a neutron capture therapy apparatus that irradiates a subject with a neutron beam. The neutron beam measuring method includes: a neutron beam generation step of generating the neutron beam; a first neutron beam irradiation step of irradiating the neutron beam generated in the neutron beam generation step toward the subject; a real-time dose output step of causing a neutron beam detection unit to output a signal in real time by detecting the neutron beam irradiated in the first neutron beam irradiation step, converting the signal into a neutron dose using a conversion condition set in advance, and outputting the neutron dose in real time; and a conversion condition setting step of setting the conversion condition. The conversion condition setting step includes a second neutron beam irradiation step of irradiating the neutron beam detection unit and a metal member with the neutron beam, a gamma ray detection step of detecting a gamma ray emitted from the metal member activated by irradiation of the neutron beam in the second neutron beam irradiation step, and a conversion condition calculation step of modifying the conversion condition based on a signal, which is output from the neutron beam detection unit by irradiation of the neutron beam, and a neutron dose of the neutron beam, which is acquired based on the gamma ray detected in the gamma ray detection step.

According to the neutron beam measuring method of the disclosure, the neutron beam detection unit detects the neutron beam and outputs a signal in real time, and the signal is converted into the neutron dose using the conversion condition in the real-time dose output step. Then, the neutron dose is output in real time. When irradiating the neutron beam to perform treatment, the signal is output in real time and the signal is converted into the neutron dose in real time, and thus, the neutron dose can be output in real time. In the conversion condition calculation step, the conversion condition is modified (updated) based on the signal, which is output from the neutron beam detection unit that detects the neutron beam, and the neutron dose acquired based on the gamma rays detected from the metal member in the gamma ray detection step. Thus, by modifying the conversion condition using the signal output from the neutron beam detection unit and the neutron dose obtained from the metal member, the signal is corrected into the neutron dose under the modified conversion condition even if the detection efficiency of the neutron beam detection unit that outputs the signal is lowered. Therefore, it is possible to eliminate a difference between the neutron dose actually irradiated and the measured neutron dose. By converting the signal into the neutron dose under the modified conversion condition and outputting the signal, it is possible to avoid a situation where the measurement accuracy of neutron beams is reduced.

According to the invention, it is possible to measure the neutron dose in real time while suppressing a reduction in the measurement accuracy of neutron beams.

### BRIEF DESCRIPTION OF THE DRAWINGS

Fig. 1 is a diagram showing the arrangement of a neutron capture therapy apparatus according to the present embodiment.
Fig. 2 is a diagram showing the vicinity of a neutron beam irradiation unit in the neutron capture therapy apparatus shown in Fig. 1.
Fig. 3 is a diagram illustrating the measurement of neutron beams in the neutron capture therapy apparatus shown in Fig. 1.
Fig. 4 is a diagram showing a scintillation detector.
Fig. 5 is a diagram illustrating the conversion condition setting process in the neutron capture therapy apparatus shown in Fig. 1.
Fig. 6 is a diagram illustrating the conversion condition setting process in the neutron capture therapy apparatus shown in Fig. 1.

### DETAILED DESCRIPTION OF THE INVENTION

Hereinafter, a neutron capture therapy apparatus according to embodiments of the invention will be described in detail with reference to the accompanying diagrams.

As shown in Figs . 1 and 2, a neutron capture therapy apparatus 1 that performs cancer treatment using a boron neutron capture therapy is an apparatus that performs cancer treatment by irradiating a part where boron is accumulated of a patient (subject) S, towhomboron (¹⁰B) has been administered, with neutron beams. The neutron capture therapy apparatus 1 includes an irradiation chamber 2 for performing cancer treatment of the patient S restrained on a treatment table 3 by irradiating the patient S with a neutron beam N. A camera 4 for imaging the patient S is disposed in the irradiation chamber 2. The camera 4 is a CCD camera, for example.

A preparatory work, such as restraining the patient S on the treatment table 3, is performed in a preparation room (not shown) outside the irradiation chamber 2, and the treatment table 3 on which the patient S is restrained is moved from the preparation room to the irradiation chamber 2. The neutron capture therapy apparatus 1 includes a neutron beam generation unit 10 that generates the neutron beam N for treatment and a neutron beam irradiation unit 20 that irradiates the patient S, who is restrained on the treatment table 3 in the irradiation chamber 2, with the neutron beam N.

The neutron beam generation unit 10 includes a cyclotron 11 that generates a charged particle beam L, a beam transport path 12 for transporting the charged particle beam L generated by the cyclotron 11, a charged particle beam scanning unit 13 that scans the charged particle beam L to control the irradiation of the charged particle beam L to a target T, and the target T. The cyclotron 11 and the beam transport path 12 are disposed in a charged particle beam generation chamber 14 having an approximately rectangular shape, and the charged particle beam generation chamber 14 is a closed space covered with a shielding wall W formed of concrete. The charged particle beam scanning unit 13 controls the irradiation position of the charged particle beam L on the target T.

The cyclotron 11 is an accelerator that accelerates charged particles, such as protons, to generate the charged particle beam L, such as a proton beam. The cyclotron 11 can generate the charged particle beam L of 60 kW (= 30 MeV x 2 mA) having a beam radius of 40 mm, for example. Instead of the cyclotron 11, other accelerators, such as a synchrotron, a synchrocyclotron, and a linac, may be used.

One end of the beam transport path 12 is connected to the cyclotron 11. The beam transport path 12 includes a beam adjusting unit 15 for adjusting the charged particle beam L. The beam adjusting unit 15 includes horizontal steering and horizontal and vertical steering for adjusting the axis of the charged particle beam L, a quadrupole electromagnet for suppressing the divergence of the charged particle beam L, and a four-way slit for shaping the charged particle beam L. The beam transport path 12 preferably has a function of transporting the charged particle beam L, and may not have the beam adjusting unit 15.

The charged particle beam L transported by the beam transport path 12 is irradiated to the target T by controlling the irradiation position using the charged particle beam scanning unit 13. The charged particle beam scanning unit 13 maybe omitted, so that the charged particle beam L is always irradiated to the same position of the target T. The target T generates the neutron beam N by the irradiation of the charged particle beam L. The target T is formed of beryllium (Be), lithium (Li), tantalum (Ta), or tungsten (W), and has a disc shape with a diameter of 160 mm, for example. The neutron beam N generated by the target T is irradiated toward the patient S in the irradiation chamber 2 by the neutron beam irradiation unit 20.

The neutron beam irradiation unit 20 includes a moderator 21 for decelerating the neutron beam N emitted from the target T and a shield 22 for shielding a radiation, such as the neutron beam N and gamma rays, so as not to be discharged to the outside. A moderator is formed by the moderator 21 and the shield 22.

The moderator 21 is a laminated structure formed of a plurality of different materials, for example, and the material of the moderator 21 is appropriately selected depending on various conditions, such as the energy of the charged particle beam L. Specifically, for example, when the output from the cyclotron 11 is a proton beam of 30 MeV and a beryllium target is used as the target T, lead, iron, aluminum, or calcium fluoride can be used as materials of the moderator 21. When the output from the cyclotron 11 is a proton beam of 11 MeV and a beryllium target is used as the target T, heavy water (D20) or lead fluoride can be used as materials of the moderator 21.

The shield 22 is provided so as to surround the moderator 21, and has a function of shielding a radiation, such as the neutron beam N and gamma rays generated due to the generation of the neutron beam N, so as not to be discharged to the outside of the shield 22. At least a part of the shield 22 is embedded in the wall W1 that separates the charged particle beam generation chamber 14 from the irradiation chamber 2. A wall 23 that forms a part of the side wall surface of the irradiation chamber 2 is provided between the irradiation chamber 2 and the shield 22. A collimator mounting portion 23a as an output port of the neutron beam N is provided in the wall 23. A collimator 31 for defining the irradiation field of the neutron beam N is fixed to the collimator mounting portion 23a.

In the neutron beam irradiation unit 20 described above, the charged particle beam L is irradiated to the target T, and the target T generates the neutron beam N. The neutron beam N generated by the target T is decelerated when passing through the moderator 21, and the neutron beam N emitted from the moderator 21 is irradiated to the patient S on the treatment table 3 after passing the collimator 31. As the neutron beam N, it is possible to use an epithermal neutron beam or a thermal neutron beam with relatively low energy.

The treatment table 3 serves as a mounting table used in the neutron capture therapy, and can restrains the patient S at a predetermined posture and move from the preparation room (not shown) to the irradiation chamber 2 with the restrained posture. The treatment table 3 includes a base portion 32 that forms the foundation of the treatment table 3, a caster 33 that can move the base portion 32 on the floor, a top plate 34 on which the patient S is placed, and a robot arm 35 for moving the top plate 34 relative to the base portion 32.

The base portion 32 includes a rectangular parallelepiped foundation portion 32a and a rectangular parallelepiped support portion 32b that is located above the foundation portion 32a and supports the robot arm 35. The support portion 32b is located inside the foundation portion 32a in plan view, and the robot arm 35 is fixed to the upper surface of the support portion 32b. The four casters 33 are attached to the lower portion of the foundation portion 32a. A driving force, such as a motor, may be applied to the casters 33. In this case, since it is possible to make the caster 33 roll easily on the floor, the movement of the treatment table 3 in a horizontal direction becomes easy.

The robot arm 35 is for moving the top plate 34 relative to the base portion 32, and can move the patient S restrained on the top plate 34 relative to the collimator 31. The top plate 34 has an approximately rectangular shape in plan view, and the length of the top plate 34 in the longitudinal direction is a length (for example, 2 m) allowing the patient S to lie down on the top plate 34. The position of the top plate 34 with respect to the base portion 32 can be adjusted in a three-dimensional manner by the movement and rotation of the robot arm 35. In addition, a restraint (not shown) for restraining the patient S on the top plate 34 is provided in the top plate 34.

The neutron beam N having passed through an opening 31a of the collimator 31 is irradiated to the patient S restrained on the top plate 34. The collimator 31 has, for example, an approximately rectangular parallelepiped shape, and includes the opening 31a for defining the irradiation range of the neutron beam N at the center. The outer shape of the collimator 31 corresponds to the inner surface shape of the collimator mounting portion 23a in the wall 23, and the collimator 31 is fixed in a state of being fitted into the collimator mounting portion 23a of the wall 23.

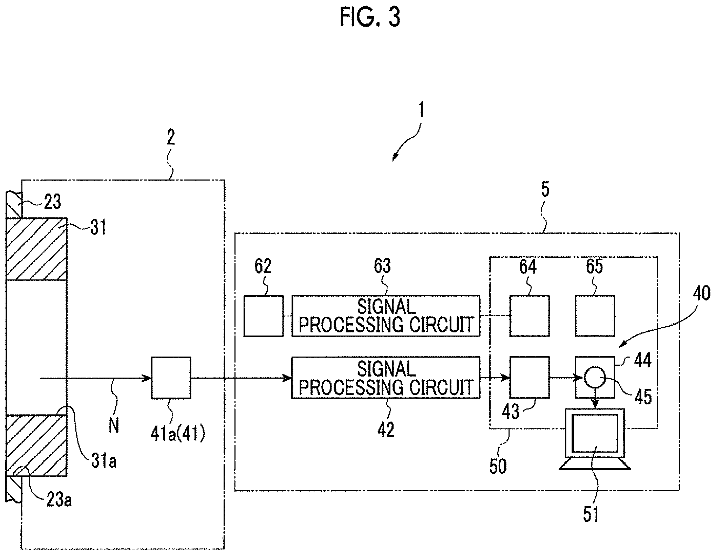

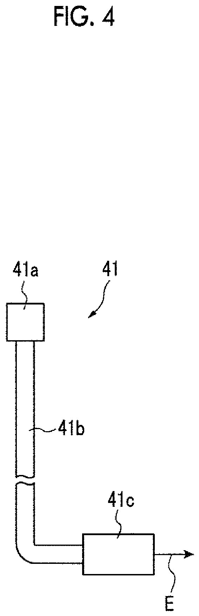

Incidentally, as shown in Fig. 3, in the neutron capture therapy apparatus 1, the neutron dose of the neutron beam N is output to a display 51 of a computer 50 in a monitoring room 5, which is a separate room from the irradiation chamber 2, in real time simultaneously with the irradiation of the neutron beam N to the patient S. Some of neutron beams irradiated from the neutron beam irradiation unit 20 are irradiated to a scintillation detector (neutron beam detection unit) 41, and the other neutron beams are irradiated to the patient S. A scintillator 41a of the scintillation detector 41 shown in Fig. 4 is affixed to the head of the patient S, for example. The scintillator 41a is a phosphor that converts the neutron beam, which is incident on the scintillator 41a, into light. According to the dose of incident neutron beams, internal crystal becomes excited, thereby generating scintillation light.

The scintillation detector 41 includes the scintillator 41a described above, a light guide 41b for transmitting light from the scintillator 41a, and a photodetector 41c that outputs an electrical signal E by performing photoelectric conversion of the light transmitted by the light guide 41b. The light guide 41b is formed of, for example, a bundle of flexible optical fibers, and transmits the light generated by the scintillator 41a to the photodetector 41c. The light guide 41b extends, for example, from the irradiation chamber 2 to the monitoring room 5, and is connected to the photodetector 41c in the monitoring room 5. The photodetector 41c detects light from the light guide 41b, and outputs the electrical signal to a signal processing circuit 42 as a pulse signal. As the photodetector 41c, for example, a phototube or a photomultiplier tube can be used.

The signal processing circuit 42 has a function of shaping the pulse signal from the scintillation detector 41 and out putting the result to a neutron beam measuring unit 43 and a noise removal function, and is disposed at a position adjacent to the computer 50 in the monitoring room 5, for example. The neutron beam measuring unit 43 is software in the computer 50, and measures the amount of neutron beam N irradiated to the scintillator 41a in real time by counting the pulses of the pulse signal received from the signal processing circuit 42.

The neutron beam measuring unit 43 measures the amount of neutron beam N as the number of signals that the scintillation detector 41 outputs per unit time (count rate). That is, the neutron beam measuring unit 43 measures data regarding the signal of the scintillation detector 41 (hereinafter, referred to as signal data), and outputs the measured signal data of the scintillation detector 41 to a neutron dose output unit 44. A real-time dose output unit 4a is formed by the scintillation detector 41, the signal processing circuit 42, the neutron beam measuring unit 43, and the neutron dose output unit 44 described above.

In the scintillation detector 41, since the optical fiber or the like of the light guide 41b deteriorates with an increase in the number of times of irradiation of the neutron beam N, the detection efficiency of the neutron beam N is reduced. If the detection efficiency of the neutron beam N is reduced as described above, signal data from the scintillation detector 41 with respect to the amount of neutron beam N actually irradiated to the scintillation detector 41 changes gradually. This causes a problem in that the measurement accuracy of the neutron beam N is reduced.

As a method of measuring the neutron beam N, for example, there is a method of measuring the amount of irradiated neutron beam N by mounting the gold wire on the head of the patient S and measuring the amount of activation of the gold wire. Thus, the method of measuring the amount of the neutron beam N by measuring the amount of activation of gold wire is a method that cannot disturb the neutron field easily. By measuring the amount of gamma rays emitted from the gold wire, it is possible to reliably detect the neutron dose. However, this method has a problem in that it takes time for the activation of gold wire and real-time measurement cannot be performed since the amount of neutron beam N cannot be measured during the irradiation of the neutron beam N. In addition, since the total amount of the dose of neutron beam N is calculated from the amount of activation of gold wire afterward, the average value of the dose per unit time can be calculated by dividing the total amount of the dose by the irradiation time. However, there is also a problem in that a change in dose per predetermined time cannot be calculated.

Therefore, in order to solve the problems described above, in the neutron capture therapy apparatus 1, the real-time dose output unit 40 can output the neutron dose in real time. In addition, the method of measuring the neutron beam N using the neutron capture therapy apparatus 1 includes a real-time dose output step of outputting the neutron dose in real time. That is, when the signal data of the scintillation detector 41 is received from the neutron beam measuring unit 43, the neutron dose output unit 44 of the real-time dose output unit 40 converts the signal data into the neutron dose of the neutron beam N using a correction coefficient 45 set in advance and outputs the dose of the neutron beam N in real time.

The correction coefficient 45 functions as a conversion condition for converting the signal of the scintillation detector 41 into the dose of the neutron beam N irradiated to the patient S, and shows the relationship between the signal data of the scintillation detector 41 and the dose of the neutron beam N irradiated to the patient S. The correction coefficient 45 is set in advance in calibration work (conversion condition setting step) before performing the treatment. Here, "before performing the treatment" indicates "before each treatment". Alternatively, calibration may be performed every predetermined number of times of treatment instead of the "before each treatment", or calibration may be performed after a predetermined time elapses. Hereinafter, this calibration work and a method of measuring the neutron beam N when treating the patient S using the neutron beam N will be described.

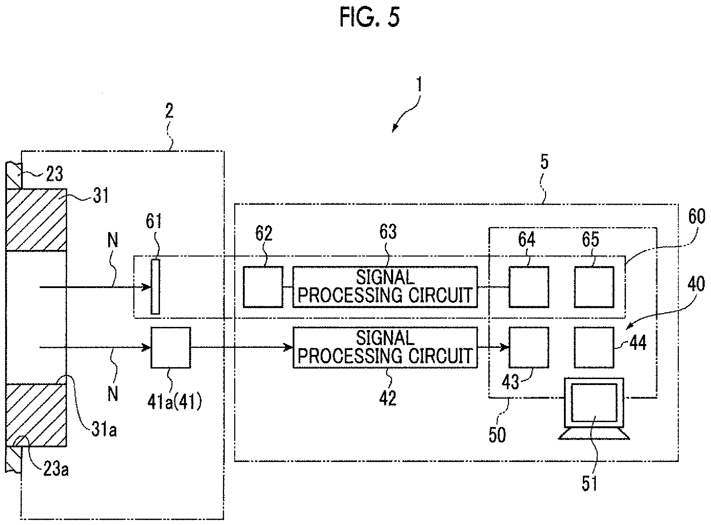

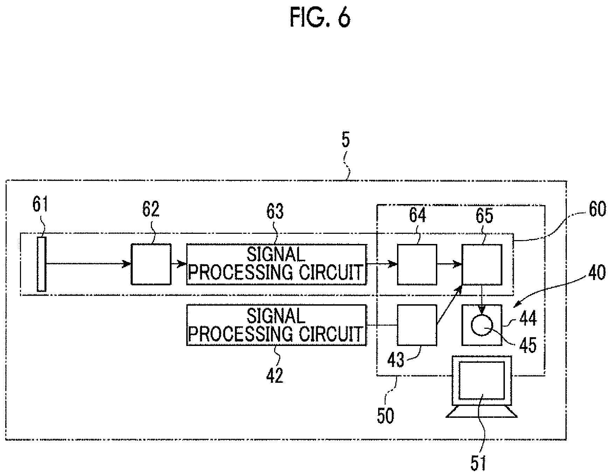

The calibration work is performed when the patient S is not present in the irradiation chamber 2, for example. As shown in Figs. 5 and 6, in the calibration work, a correction coefficient setting unit (conversion condition setting unit) 60 sets the correction coefficient 45 before treatment. The correction coefficient setting unit 60 includes a gold wire (metal member) 61 that is a thin wire of gold (Au), a gamma ray detector (gamma ray detection section) 62 that detects gamma rays, a signal processing circuit 63 that processes a signal from the gamma ray detector 62, a neutron dose measurement section 64 that measures the dose of the neutron beam N, and a correction coefficient calculation section (conversion condition calculation section) 65 that calculates the correction coefficient 45.

As shown in Fig. 5, the scintillation detector 41 is disposed at a position where the head of the patient S is located, for example. The gold wire 61 is disposed adjacent to the scintillation detector 41. The scintillation detector 41 and the gold wire 61 are disposed such that their positions and directions with respect to the opening 31a of the collimator 31 are the same. Under such conditions, the scintillation detector 41 and the gold wire 61 are irradiated with the neutron beam N for a fixed period of time (for example, 10 minutes) (second neutron beam irradiation step). When the scintillation detector 41 is irradiated with the neutron beam N for a fixed period of time, the neutron beam measuring unit 43 measures the signal data of the scintillation detector 41 in real time.

As shown in Fig. 6, after the neutron beam N is irradiated for a fixed period of time, the irradiation of the neutron beam N to the scintillation detector 41 and the gold wire 61 is stopped. Then, the gold wire 61 activated by irradiation of the neutron beam N is taken away from the irradiation chamber 2 , and is disposed in the holder of the monitoring room 5. After placing the gold wire 61 in the holder, the correction coefficient 45 is calculated by the gamma ray detector 62, the signal processing circuit 63, the neutron dose measurement section 64, and the correction coefficient calculation section 65. The process of calculating the correction coefficient 45 is automatically performed when the computer 50 is operated after the gold wire 61 is disposed.

The gamma ray detector 62 detects gamma rays generated from the activated gold wire 61 (gamma ray detection step). As the gamma ray detector 62, a semiconductor detector, such as a germanium detector, can be used. For example, when gamma rays are incident on the semiconductor crystal of the gamma ray detector 62, an electrical signal is generated. The gamma ray detector 62 outputs the electrical signal to the signal processing circuit 63. The gamma ray detector 62 and the signal processing circuit 63 are disposed at positions adjacent to the computer 50 in the monitoring room 5, for example.

The signal processing circuit 63 has a function of shaping the electrical signal from the gamma ray detector 62 and outputting the result to the neutron dose measurement section 64 and a noise removal function. The neutron dose measurement section 64 receives the electrical signal shaped by the signal processing circuit 63 and measures the dose of the neutron beam N irradiated to the gold wire 61. The neutron dose measurement section 64 measures the amount of neutron beam N as the amount of neutron beam N per unit area, that is, a neutron flux. The unit of the neutron dose measured by the neutron dose measurement section 64 is N/cm². The dose of the neutron beam N measured by the neutron dose measurement section 64 is output to the correction coefficient calculation section 65.

The dose of the neutron beam N measured by the neutron dose measurement section 64 and the signal data of the scintillation detector 41 measured by the neutron beam measuring unit 43 are input to the correction coefficient calculation section 65. Then, the correction coefficient calculation section 65 calculates the correction coefficient 45 for matching the signal of the scintillation detector 41 and the neutron dose with each other from the signal data of the scintillation detector 41 and the dose of the neutron beam N (conversion condition calculation step). After the correction coefficient 45 is calculated by the correction coefficient calculation section 65 as describedabove, a series of calibration work is completed. The correction coefficient 45 calculated herein is a neutron dose, which is measured by the neutron dose measurement section 64 , with respect to the unit data (the number of light beams per unit time) measured by the neutron beam measuring unit 43.

After the calibration work is completed as described above, a preparatory work, such as restraining the patient S on the treatment table 3, is performed. After moving the treatment table 3 on which the patient S is restrained into the irradiation chamber 2, alignment of the patient S with respect to the collimator 31 is performed by driving the robot arm 35, so that the head of the patient S is close to the opening 31a of the collimator 31 and the irradiation position of the neutron beam N is located in the affected area of the patient S. Then, the patient S is irradiated with the neutron beam N to start the treatment.

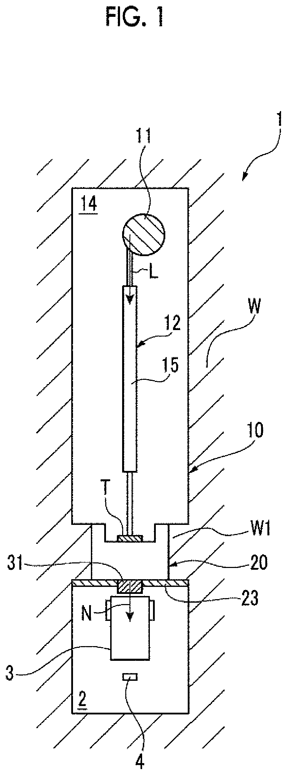

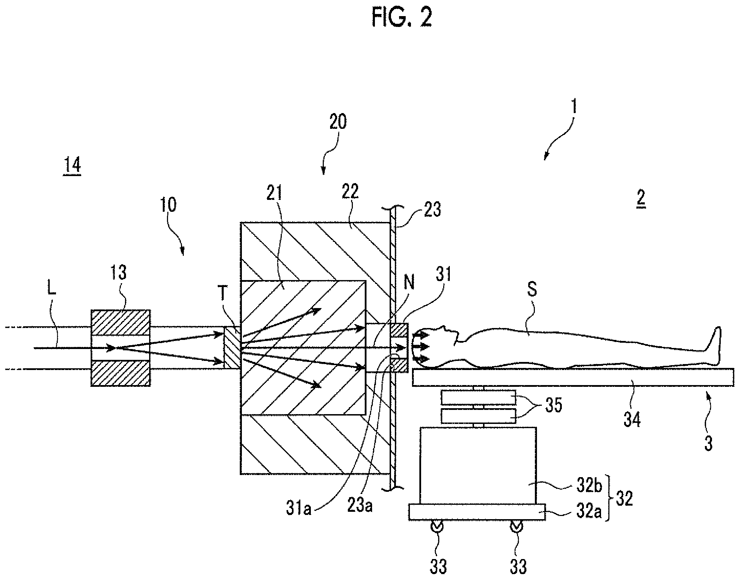

Specifically, as shown in Fig. 1, the cyclotron 11 generates the charged particle beam L, and the charged particle beam L is irradiated to the target T through the beam transport path 12 and the charged particle beam scanning unit 13. Then, as shown in Fig. 2, the target T generates the neutron beam N (neutron beam generation step). The neutron beam N generated by the target T is decelerated by the moderator 21, and is then irradiated to the patient S after the irradiation range is defined by the opening 31a of the collimator 31 (first neutron beam irradiation step).

As shown in Fig. 3, the neutron beam N irradiated from the collimator 31 is incident on the scintillation detector 41. When the neutron beam N is incident on the scintillation detector 41, the signal data of the scintillation detector 41 is transmitted to the neutron beam measuring unit 43 through the signal processing circuit 42 in real time. Then, the signal data of the scintillation detector 41 is converted into the dose of the neutron beam N using the correction coefficient 45 by the neutron dose output unit 44, and is output to the display 51 of the computer 50 in real time (real-time dose output step). Thus, since the dose of the neutron beam N is output to the display 51 in real time, a doctor or the like can always see the dose of the neutron beam N during treatment.

As described above, according to the neutron capture therapy apparatus 1 and the neutron beam measuring method using the neutron capture therapy apparatus 1, the scintillation detector 41 detects the neutron beam N irradiated from the neutron beam irradiation unit 20 and outputs the signal in real time, the neutron dose output unit 44 converts the signal data into a neutron dose using the correction coefficient 45 set in advance, and the neutron dose is output to the display 51 of the computer 50 in real time. Thus, due to the real-time dose output unit 40 including the scintillation detector 41 and the neutron dose output unit 44, the neutron dose of the neutron beam N can be output in real time when irradiating the neutron beam N to perform treatment.

The correction coefficient setting unit 60 includes the gamma ray detector 62 that detects gamma rays emitted from the gold wire 61 activated by irradiation of the neutron beam N, and modifies (updates) the correction coefficient 45 based on the signal data, which is output from the scintillation detector 41 that detects the neutron beam N, and the neutron dose acquired based on the gamma rays detected by the gamma ray detector 62. By modifying the correction coefficient 45 using the signal data, which is output from the scintillation detector 41 that detects the neutron beam N, and the neutron dose obtained from the gold wire 61, the signal data of the scintillation detector 41 is corrected into the neutron dose using the modified correction coefficient 45 even if the detection efficiency of the scintillation detector 41 that outputs a signal is lowered. That is, even if the detection efficiency of the scintillation detector 41 is lowered, it is possible to accurately calculate the neutron dose by resetting the correction coefficient 45. Therefore, it is possible to eliminate a difference between the neutron dose actually irradiated and the measured neutron dose. Thus, since the signal data of the scintillation detector 41 is output after being converted into the neutron dose using the modified correction coefficient 45, it is possible to avoid a situation where the measurement accuracy of neutron beams is reduced.

In the neutron capture therapy apparatus 1, the scintillator 41a that generates a light signal when neutron beams are irradiated is used as a neutron beam detection unit. Since the scintillator 41a can be easily miniaturized and can be easily processed into various shapes, it is possible to reduce the size of the apparatus by using the scintillator 41a.

The neutron beam measuring unit 43 , the neutron dose output unit 44, the neutron dose measurement section 64, and the correction coefficient calculation section 65 are software in the computer 50, and the signal processing circuit 42, the gamma ray detector 62 , and the signal processing circuit 63 are disposed at positions adjacent to the computer 50. Thus, each device to perform calibration work or measurement work of the neutron beam N is collected within the monitoring room 5. The calibration work or the measurement work of the neutron beam N is automatically performed by each device in the monitoring room 5 when a setting of the scintillation detector 41 or the gold wire 61 is performed. Thus, the neutron capture therapy apparatus 1 has a configuration to easily perform the calibration work or the measurement work of the neutron beam N.

While the embodiments of the invention have been described above, the invention is defined in the claims. Other embodiments are merely examplary.

In the embodiment described above, the calibration work is performed when the patient S is not present in the irradiation chamber 2. However, the timing of the calibration work can be appropriately changed. For example, the calibration work may be performed during treatment for which the patient S is present in the irradiation chamber 2. That is, assuming that the entire irradiation time is 30 minutes, it is possible to perform calibration work using the gold wire 61 in the first 10 minutes and perform real-time measurement of the neutron dose in the subsequent 20 minutes.

In addition, although the gold wire 61 is used as a metal member of the correction coefficient setting unit 60, it is also possible to use a metal member having a different shape from the gold wire 61, for example, a gold leaf. Materials of the metal member are not limited to gold, and it is possible to use any material that is activated when neutron beams are irradiated. For example, manganese can be used.

In the embodiment described above, the correction coefficient calculation section 65 calculates the correction coefficient 45 from the signal data of the scintillation detector 41 and the dose of the neutron beam N. However, it is also possible to calculate the correction coefficient 45 from those other than the signal of the scintillation detector 41. For example, in the second and subsequent calculations of the correction coefficient 45, the correction coefficient 45 can be calculated from the neutron dose output from the neutron dose output unit 44 last and the neutron dose obtained this time by the neutron dose measurement section 64. As a conversion condition for converting the signal data into the neutron dose, for example, an equation and the like can be used without being limited to the correction coefficient 45.

In addition, although the scintillation detector 41 is used as a neutron beam detection unit that receives neutron beams and generates a signal in real time, an ionization chamber may be used instead of the scintillation detector 41.

In the monitoring room 5, the computer 50, the gamma ray detector 62, and the signal processing circuits 42 and 63 are disposed at positions adjacent to each other. However, these arrangement positions maybe appropriately changed. In addiction, it is also possible to appropriately change the configuration of the neutron beam generation unit 10 and the neutron beam irradiation unit 20 or the arrangement of each device in the neutron capture therapy apparatus 1.

## Claims

1. A neutron capture therapy apparatus (1) adapted to irradiate a subject (S) with a neutron beam (N), comprising:
a neutron beam generation unit (10) adapted to generate the neutron beam (N);
a neutron beam irradiation unit (20) adapted to irradiate the neutron beam (N) generated by the neutron beam generation unit (10) toward the subject (S);
a real-time dose output unit (40) that includes a neutron beam detection unit (41), being adapted to detect the neutron beam (N) irradiated from the neutron beam irradiation unit (20) and to output a signal in real time, and a neutron dose output unit (44), adapted to convert the signal output from the neutron beam detection unit (41) into a neutron dose using a conversion condition set in advance and outputs the neutron dose in real time;
**characterized by** further comprising:
a metal member (61);
a conversion condition setting unit (60) adapted to set the conversion condition,
wherein the conversion condition setting unit (60) includes a gamma ray detection section (62) adapted to detect a gamma ray emitted from said metal member (61) activated by irradiation of the neutron beam (N), and to modify the conversion condition set in advance based on a signal, which is output from the neutron beam detection unit (41) by irradiation of the neutron beam (N), and a neutron dose of the neutron beam (N), which is acquired based on the gamma ray detected by the gamma ray detection section (62).

2. The neutron capture therapy apparatus (1) according to claim 1,
wherein the neutron beam detection unit is a scintillator (41) adapted to generate a light signal as the signal by irradiation of the neutron beam (N).

## Patentansprüche

1. Neutroneneinfangtherapie-Vorrichtung (1), die zur Bestrahlung eines Subjekts (S) mit einem Neutronenstrahl (N) geeignet ist, umfassend:
eine Neutronenstrahl-Erzeugungseinrichtung (10), die zur Erzeugung von Neutronenstrahlen (N) geeignet ist;
eine Neutronenstrahl-Bestrahlungseinrichtung (20), die geeignet ist, den von der Neutronenstrahl-Erzeugungseinrichtung (10) erzeugten Neutronenstrahl (N) auf das Subjekt (S) zu richten;
eine Echtzeit-Dosisausgabeeinrichtung (40), die eine Neutronenstrahl-Erfassungseinrichtung (41) umfasst und dazu geeignet ist, den von der Neutronenstrahl-Bestrahlungseinrichtung (20) ausgestrahlten Neutronenstrahl (N) zu erfassen und ein Signal in Echtzeit auszugeben; und
eine Neutronen-Dosisausgabeeinrichtung (44), die dazu geeignet ist, das von der Neutronenstrahl-Erfassungseinrichtung (41) ausgegebene Signal unter Anwendung einer vorher festgelegten Umwandlungsbedingung in eine Neutronendosis umzuwandeln und die Neutronendosis in Echtzeit auszugeben;
**dadurch gekennzeichnet, dass** die Vorrichtung ferner Folgendes umfasst:
ein Metallbauteil (61);
eine Umwandlungsbedingung-Einstelleinrichtung (60), die zum Einstellen der Umwandlungsbedingung geeignet ist,
wobei die Umwandlungsbedingung-Einstelleinrichtung (60) einen gamma-Strahlen-Erfassungsbereich (62) umfasst, der dazu geeignet ist, einen vom Metallbauteil (61), das durch Bestrahlung mit dem Neutronenstrahl (N) aktiviert worden ist, emittierten gamma-Strahl zu erfassen und die vorher festgelegte Umwandlungsbedingung auf der Grundlage eines Signals, das von der Neutronenstrahl-Erfassungseinrichtung (41) durch Bestrahlen mit dem Neutronenstrahl (N) ausgegeben worden ist, und einer Neutronendosis des Neutronenstrahls (N), die auf der Grundlage des durch den gamma-Strahlen-Erfassungsbereich (62) erfassten gamma-Strahls ermittelt worden ist, zu modifizieren.

2. Neutroneneinfangtherapie-Vorrichtung (1) nach Anspruch 1,
wobei es sich bei der Neutronenstrahl-Erfassungseinrichtung um einen Szintillator (41) handelt, der dazu geeignet ist, ein Lichtsignal als das Signal durch Bestrahlung mit dem Neutronenstrahl (N) zu erzeugen.

## Revendications

1. Appareil de thérapie par capture de neutrons (1) conçu pour irradier un sujet (S) avec un faisceau de neutrons (N), comprenant :
une unité de génération de faisceau de neutrons (10) conçue pour générer le faisceau de neutrons (N) ;
une unité d'irradiation de faisceau de neutrons (20) conçue pour irradier le faisceau de neutrons (N) généré par l'unité de génération de faisceau de neutrons (10) vers le sujet (S) ;
une unité de production de dose en temps réel (40) qui comprend une unité de détection de faisceau de neutrons (41), conçue pour détecter le faisceau de neutrons (N) irradié à partir de l'unité d'irradiation de faisceau de neutrons (20) et pour produire un signal en temps réel, et une unité de production de dose de neutrons (44), conçue pour convertir le signal produit à partir de l'unité de détection de faisceau de neutrons (41) en une dose de neutrons au moyen d'un ensemble de conditions de conversion à l'avance et produit la dose de neutrons en temps réel;
**caractérisé en ce qu'**il comprend en outre :
un élément métallique (61) ;
une unité de définition de conditions de conversion (60) conçue pour définir la condition de conversion,
dans lequel l'unité de définition de conditions de conversion (60) comprend une section de détection de rayons gamma (62) conçue pour détecter un rayon gamma émis à partir dudit élément métallique (61 activé par l'irradiation du faisceau de neutrons (N), et pour modifier l'ensemble de conditions de conversion à l'avance sur la base d'un signal, qui est produit à partir de l'unité de détection de faisceau de neutrons (41) par irradiation du faisceau de neutrons (N), et une dose de neutrons du faisceau de neutrons (N), qui est acquise sur la base du rayon gamma détecté par la section de détection de rayons gamma (62).

2. Appareil de thérapie par capture de neutrons (1) selon la revendication 1, dans lequel l'unité de détection de faisceaux de neutrons est un scintillateur (41) conçu pour générer un signal lumineux en tant que signal par irradiation du faisceau de neutrons (N).
